# NEUE EUROPÄISCHE PATENTSCHRIFT

(11) **EP 1 213 974 B2**
(45) Veröffentlichungstag und Bekanntmachung des Hinweises auf die Entscheidung über den Einspruch: **08.08.2012**
(45) Hinweis auf die Patenterteilung: 21.12.2005
(21) Anmeldenummer: 00962465.1
(22) Anmeldetag: 09.09.2000
(51) Int. Cl.: A23L 1/236, A23G 3/00, A23P 1/02, C07H 15/04

(54) **DIREKT VERPRESSBARER ROHSTOFF FÜR KOMPRIMATE**
DIRECTLY COMPRESSABLE RAW MATERIAL FOR TABLETS
MATIERE PREMIERE DIRECTEMENT COMPRIMABLE POUR COMPRIMES

(30) Priorität: 10.09.1999 DE 19943496
(43) Veröffentlichungstag der Anmeldung: 19.06.2002
(73) Patentinhaber: Südzucker Aktiengesellschaft Mannheim/Ochsenfurt, 68165 Mannheim (DE)
(72) Erfinder: BAYERKÖHLER, Theodor, 68165 Mannheim (DE); DEGELMANN, Hanspeter, 67549 Worms (DE); DÖRR, Tillmann, 67591 Hohen-Sülzen (DE); GUDERJAHN, Lutz, D-67591 Offstein (DE); JANSSEN, Holger, 67591 Hohen-Sülzen (DE); KOWALCZYK, Jörg, 67248 Bockenheim (DE)
(74) Vertreter: Schrell, Andreas
(86) Internationale Anmeldenummer: PCT/EP2000/008830
(87) Internationale Veröffentlichungsnummer: WO 2001/019208

(56) Entgegenhaltungen:
- WO-A-00/64916
- WO-A-90/14821
- WO-A-99/47532
- WO-A1-98//12936
- DE-A1- 1 941 956
- DE-A1- 3 732 141
- JP-A- 60 163 893
- US-A- 4 572 916
- US-A- 5 616 361

## Beschreibung

Die vorliegende Erfindung betrifft ein Verfahren zur Herstellung eines agglomerierten, fließfähigen Produktes aus Isomaltulose, daraus hergestellte Komprimate sowie Verfahren zur Herstellung der Agglomerate und Komprimate.

Komprimate sind aus zusammengepreßten Bestandteilen bestehende Genuß-, Arznei- oder auch Nahrungsmittel. Komprimate enthalten dementsprechend im allgemeinen ein Träger- oder Verdünnungsmittel, Bindemittel, Trenn- oder Gleitmittel sowie die aktiven Wirkstoffe, wie Geschmacksstoffe, Arzneistoffe oder Süßungsmittel. Als Träger- beziehungsweise Verdünnungsmittel wird häufig Saccharose, Lactose, Glucose, Stärke oder Mannit verwendet.

Die EP-B1 0 028 905 offenbart Isomaltulose enthaltende Tabletten und Verfahren zu deren Herstellung. Die Druckschrift offenbart eine vorteilhafte Verwendung von Isomaltulose als Verdünnungsmittel für die Komprimatherstellung, da Isomaltulose direkt ohne Bindemittel und ohne kontrollierte Granulierung verpreßt werden könne. Gemäß dieser Druckschrift wird direkt aus der enzymatischen Umwandlung von Saccharose zu Isomaltulose hergestellte kristallisierte Isomaltulose für die Tablettierung verwendet.

Die DE 196 39 343 C2 offenbart Isomalt und Isomalt-Varianten enthaltende Komprimate. Die Herstellung der Komprimate erfolgte durch einfaches Verpressen der Einzelkomponenten ohne eine spezielle mechanische und/oder chemische Behandlung der Einzelkomponenten vorzusehen.

Aus der EP-A1 0 625 578 gehen Isomalt-Varianten hervor, jedoch keine Komprimate, die diese Süßungsmittel enthalten.

Die im Stand der Technik bekannten Isomaltulose-, Isomalt- und Isomalt-Varianten-haltigen Komprimate zeichnen sich allesamt durch die Notwendigkeit aus, vergleichsweise hohe Preßdrücke bei der Komprimat-Herstellung einzusetzen, wobei vergleichsweise nur geringe Tablettenhärten erzielt werden können. Zudem sind die Komprimate des Standes der Technik hinsichtlich ihrer sensorischen Eigenschaften verbesserungsfähig, zeigen zum Beispiel Rauhigkeit beim Zerbeißen sowie nachteiliges Bruchverhalten und ein verbesserungsfähiges Ablutschverhalten.

Das der vorliegenden Erfindung zugrundeliegende technische Problem besteht also darin, ein Verfahren zur Herstellung von Komprimaten aus Isomaltulose bereitzustellen, welches die vorgenannten Nachteile überwindet, insbesondere in der Lage ist, unter Verwendung möglichst niedriger Preßdrücke Komprimate großer Härte herzustellen, die sich durch verbesserte sensorische Eigenschaften und ein verbessertes Bruchverhalten auszeichnen.

Die vorliegende Erfindung löst das ihr zugrundeliegende technische Problem durch die Bereitstellung eines Verfahrens zur Herstellung eines agglomerierten, getrockneten und fließfähigen Produktes aus Isomaltulose, wobei eine Isomaltulose-haltige Lösung oder Suspension mit einem Trokensubstanzgehalt von 30 bis 70 Gew.-% unter Zufuhr von Trockenluft auf beziehungsweise in ein Pulver aus Isomaltulose gesprüht wird, wobei daß Mengenverhältnis von Pulver zu Edukt Lösung oder Suspension 1:1 bis 3,5:1 beträgt wobei die Trocknungsluft eine Temperatur von 120 bis 180°C aufweist und wobei das so erhaltene agglomerierte Produkt einer Nachtrocknung und Kühlung unterzogen und ein agglomeriertes, fließfähiges und getrocknetes Produkt erhalten wird. Die Erfindung löst das ihr zugrundeliegende technische Problem auch durch die Bereitstellung eines gemäß des vorliegenden Verfahrens hergestellten fließfähigen, getrockneten Agglomerats sowie eines Verfahrens zur Herstellung eines Komprimates aus einem wie vorstehend erhaltenen fließfähigen Agglomerat, wobei dieses mit Hilfsstoffen und/oder Aromen versetzt und anschließend verpreßt wird.

Im Anschluß an die so durchgeführte Sprühtrocknung wird das so erhaltene agglomerierte Produkt einer Nachtrocknung und einer Kühlung unterzogen und ein agglomiertes, kristallines und fließfähiges Produkt erhalten. Dieses agglomerierte, fließfähige im Kontext der vorliegenden Erfindung auch als Agglomerat bezeichnete getrocknete Produkt zeichnet sich durch eine gute Löslichkeit, eine gute Streufähigkeit, ein geringes Schüttgewicht und eine gute Verpreßbarkeit aus. Es eignet sich daher in hervorragender Weise als Ausgangsstoff für die Komprimat-Herstellung und ist auch Gegenstand der Erfindung. Das erhaltene Agglomerat aus Isomaltulose, kann zum Beispiel als Süßungsmittel oder als Füllstoff (bulking agent) in zum Beispiel einem Komprimat eingesetzt werden.

Die erfindungsgemäße Sprühtrocknung kann in bevorzugter Ausführungsform der Erfindung vorteilhafterweise mittels eines Sprühbandtrockners, umfassend einen Sprühturm, der oberhalb einer beweglichen Oberfläche, zum Beispiel eines Gewebebandes, angeordnet ist, durchgeführt werden. In vorteilhafter Weise wird in einer bevorzugten Ausführungsform der Erfindung am Kopfende des Sprühturmes getrennt voneinander die Edukt-Lösung oder -Suspension und, vorzugsweise vermahlenes, insbesondere recyclisiertes, Pulver zugeführt. Im Gleichstrom dazu wird in vorteilhafter Weise die Trocknungsluft zugeführt. Das zugeführte Pulver dient als Kristallisationsgrundlage für die Edukt-Lösung oder -Suspension. Die Edukt-Lösung oder -Suspension agglomeriert also das Pulver. Das Pulver und die eingesprühte Edukt-Lösung beziehungsweise -Suspension fallen miteinander vermischt und einander benetzend sowie agglomerierend im Sprühturm herab, wobei das Wasser der Edukt-Lösung oder -Suspension verdampft und auf dem im unteren Teil des Sprühturms angeordneten Gewebeband eine feuchte Produktschicht abgeschieden wird, daß das so erhaltene agglomerierte Produkt in eine nachfolgende Nachtrocknungs- und Kühlzone transportiert. Selbstverständlich kann der Transport auch mittels zum Beispiel einer Schnecke durchgeführt werden. In der Nachtrocknungs- und Kühlzone findet eine Evaporation und Abkühlung des Agglomerats statt.

In einer besonders bevorzugten Ausführungsform der Erfindung kann vorgesehen sein, daß nach der Kühlung eine Nachkristallisation, beispielsweise in einer Kristallisationstrommel, durchgeführt wird.

Die Erfindung sieht in einer weiteren bevorzugten Ausführungsform vor, daß das nach der Kühlung gewonnene agglomerierte, getrocknete und fließfähige Produkt, gegebenenfalls nach Durchführung des Nachkristallisationsschrittes, zumindest teilweise gemahlen und in einer besonders bevorzugten Ausführungsform das gemahlene Produkt als Edukt dem vorliegenden Verfahren wieder zugeführt wird, das heißt, als Träger für die eingesprühte Edukt-Lösung oder -Suspension fungiert. Ein Teil des agglomerierten, fließfähigen Produktes wird also in Pulverform überführt und im Verfahren recyclisiert. Die Vermahlung kann in einer Mühle, beispielsweise einer Sichtermühle, geschehen. In einer besonders bevorzugten Ausführungsform der Erfindung ist vorgesehen, das Pulver auf eine Körnung von 10 bis 400 µm Partikeldurchmesser, insbesondere 30 bis 300 µm, besonders bevorzugt 50 bis 200 µm, zu vermahlen. Im Zusammenhang mit der vorliegenden Erfindung wird unter einem Partikeldurchmesser von zum Beispiel 30 bis 300 µm verstanden, daß mindestens 90 % der Partikel (d₉₀) der Fraktion einen Durchmesser von ≥ 30 bis ≤ 300 µm aufweisen.

Die Edukt-Lösung oder -Suspension, also die Isomaltulose-haltige Lösung oder -Suspension weist einen Trockensubstanzgehalt von 30 bis 70 Gew.-%, vorzugsweise 60 bis 70 Gew.-%, auf. In einer weiteren bevorzugten Ausführungsform der vorliegenden Erfindung weist die Edukt-Lösung oder -Suspension eine Temperatur von 50°C bis 90°C, insbesondere 65°C bis 80°C, auf.

In einer weiteren bevorzugten Ausführungsform der Erfindung wird die Edukt-Lösung oder -Suspension in das Pulver mit einem Sprühdruck von 100 bis 200 bar, insbesondere 120 bis 180 bar, eingesprüht.

Das Mengenverhältnis von Pulver zu Edukt-Lösung oder -Suspension beträgt 1:1 bis 3,5:1, vorzugsweise 2,5:1 bis 3,3:1.

Gemäß der Erfindung beträgt die Temperatur der Trocknungsluft 120°C bis 180°C, insbesondere 140°C bis 160°C. In einer weiteren bevorzugten Ausführungsform der vorliegenden Erfindung beträgt die Temperatur der Nachtrocknungs- und Kühlluft 20°C bis 80°C, insbesondere 20°C bis 60°C.

Die Erfindung sieht in einer weiteren bevorzugten Ausführungsform vor, das agglomerierte Produkt über einen Zeitraum von 10 bis 30 min., vorzugsweise 15 bis 25 min., in dem Nachtrocknungs- und Kühlbereich zu belassen, wobei die Produkttemperatur nach Verlassen des Kühlbereichs insbesondere in einem Bereich von 20°C bis 40°C, vorzugsweise 25°C bis 35°C, liegt. Das getrocknete Agglomerat weist einen Wassergehalt von 1,0 - 5 Gew.-%, bevorzugt 1,5 - 3,5 Gew.-% auf. Der Kristallisationsgrad beträgt vorzugsweise > 50%, besonders bevorzugt > 70% und ganz besonders bevorzugt > 90%. Dies führt in vorteilhafter Weise dazu, daß im Endprodukt auftretende plastische Eigenschaften vermieden werden, was aus sensorischen Gründen vorteilhaft ist.

Schließlich sieht die Erfindung in einer weiteren bevorzugten Ausführungsform vor, daß -sofern eine Nachkristallisation durchgeführt wird- diese über einen Zeitraum von 1 bis 4 Stunden, vorzugsweise 1,5 bis 2,5 Stunden, durchgeführt wird.

Selbstverständlich sieht die Erfindung auch vor, daß das nach dem Sprühtrocknen erhaltene agglomerierte Produkt vor, während oder nach der Nachtrocknung beziehungsweise Kühlung zerkleinert, zum Beispiel gemahlen, und/oder gesiebt wird, beispielsweise mittels einer Taumelsiebmaschine. Dadurch lassen sich Fraktionen mit genau definierten Partikelgrößen gewinnen und abtrennen und der Weiterverarbeitung oder Recyclisierung zuführen.

Die Erfindung sieht in vorteilhafter Weise in bevorzugter Ausführungsform auch vor, daß der für die erfindungsgemäße Recyclisierung vorgesehene Anteil des Agglomerats, also das gemahlene, agglomerierte und fließfähige Produkt permanent, also kontinuierlich, im Kreislauf geführt, das heißt, der eingesprühten Edukt-Lösung oder -Suspension zugeführt wird.

Im Zusammenhang mit der vorliegenden Erfindung wird unter einem Komprimat ein aus zusammengepreßten Bestandteilen bestehendes Genuß-, Arznei- oder Nahrungsmittel verstanden. Ein Komprimat im Sinne der vorliegenden Erfindung ist zum Beispiel eine Tablette. Die nach dem erfindungsgemäßen Verfahren hergestellten Agglomerate zeichnen sich im allgemeinen durch eine gute Fließfähigkeit und gute selbstbindende Eigenschaften aus, die ein Verkleben an einer Presse weitgehend oder ganz unmöglich machen.

Aus dem erfindungsgemäßen Rohstoff, also Agglomerat, hergestellte Komprimate können Hilfs- und Zusatzstoffe, wie Schmiermittel, Binder, Verdünnungsmittel sowie Aromastoffe, Geschmacksstoffe, Trennmittel, Farbstoffe, Süßungsmittel und/oder Arzneistoffe enthalten.

Im Zusammenhang mit der vorliegenden Erfindung wird unter einer Isomaltulose-haltigen Lösung oder Suspension eine Lösung oder Suspension des Eduktes in Wasser verstanden.

Die Erfindung sieht in einer weiteren vorteilhaften Ausgestaltung der vorliegenden Lehre vor, nach dem Agglomerieren, der Nachtrocknung und Kühlung, aber vor dem Verpressen des Agglomerats, eine Größenfraktionierung, insbesondere eine Abtrennung von Überkorn und Stäuben, der agglomerierten Produkte durchzuführen. Dabei kann vorzugsweise eine Siebmaschine mit einer Siebbelegung von beispielsweise 0,8 mm bis 0,1 mm vorgesehen sein.

In einem weiteren Verfahrensschritt kann erfindungsgemäß vorgesehen sein, das agglomerierte und gegebenenfalls nach der Agglomeration fraktionierte Produkt direkt zu verpressen, das heißt Komprimate herzustellen. Dabei kann vorgesehen sein, den Agglomeraten Hilfs- oder Zusatzstoffe, wie Trenn-oder Gleitmittel, Wirkstoffe, oder die nachfolgend genannten Stoffe etc., hinzuzufügen. Derartige Stoffe können Geschmacks- oder Aromastoffe, Süßstoffe, lebensmittelverträgliche Säuren, Sprengmittel, Farbstoffe, Vitamine, Mineralien, Süßstoffe, Wirkstoffe, die auch hinsichtlich der Auslobung vorteilhafter Eigenschaften gezielt eingesetzt werden können, Monosaccharide, Disaccharide, Monosaccharidalkohole, Stärke, Stärkederivate, Pektin, Polyvinylpyrrolidon, Cellulose, Cellulosederivate, Stearinsäure oder deren Salze, Inulin, Oligofructose oder functional foods sein. Alternativ können die genannten Hilfs- oder Zusatzstoffe direkt zur Eduktlösung oder Suspension gegeben und sprühgetrocknet werden, d.h. vor der Herstellung der Agglomerate dem Edukt zugeführt werden. Erfindungsgemäß kann also z.B. Inulin, besonders bevorzugt in einer Menge bis zu 30 Gew.-% dem Agglomerat oder dem Edukt zugesetzt werden. Den Agglomeraten oder dem Edukt können auch Sorbit, Mannit, hydrierte oder nicht-hydrierte Oligosaccharide, Erythrit, Xylit oder Zucker, wie Saccharose, Glucose, Lactose, Fructose oder Xylose, zugefügt werden. In vorteilhafter Weise liegt der Anteil dieser Stoffe, bezogen auf das Gesamttrockengewicht, bei einer Menge von weniger als 30 Gew.-%, vorzugsweise weniger als 25, 20, 15, 10 oder 5 Gew.-%. In einer weiteren Ausführungsform können die Komprimate Xylit-frei sein. In einer weiteren bevorzugten Ausführungsform sind die erfindungsgemäßen Komprimate für Diabetiker-geeignet, zahnfreundlich, bifidogen oder blutfett-reduzierend.

Den Agglomeraten oder dem Edukt können ferner Intensiv-Süßstoffe, wie Dipeptid-Süßstoffe, Saccharin, Acesulfam-K, Aspartam, Cyclamat, Glycyrrhizin, Taumatin, Saccharin, Steveoside, Neohesperidin-Dihydrochalkon und/oder Sucralose zugefügt werden. In vorteilhafter Weise enthalten die erfindungsgemäßen Komprimate zudem Geschmacks- oder Aromastoffe, wie Citronen- oder Pfefferminz-Aroma. Die erfindungsgemäßen Komprimate können auch lebensmittelverträgliche Säuren, wie Ascorbinsäure oder Citronensäure, sowie als Gleitmittel Fettsäuren oder deren Salze, wie Magnesiumstearat oder Natriumstearat, enthalten. Schließlich kann vorgesehen sein, daß in den erfindungsgemäßen Komprimaten Farbstoffe und/oder Sprengmittel, wie Bicarbonat oder Carboxymethylcellulose, enthalten sind.

In einer besonders bevorzugten Ausführungsform ist vorgesehen, Komprimate bereitzustellen, die pharmazeutisch aktive Wirkstoffe in den Mund- und Rachenraum bringen und dort freisetzen können. Im Zusammenhang mit der vorliegenden Erfindung sind unter pharmazeutisch aktiven Wirkstoffen Substanzen zu verstehen, die einen erwünschten prophylaktischen oder therapeutischen Effekt auf den menschlichen oder tierischen Körper haben. Diese Substanzen dienen also insbesondere der Prophylaxe oder Therapie von Mangelzuständen oder Krankheitsbildern. Erfindungsgemäß können beispielsweise Enzyme, Coenzyme, Mineralstoffe, Vitamine, Antibiotica, microbizid oder fungizid wirkende Stoffe, Nikotin, Coffein, Zink, Eukalyptus, Menthol, Codein, Phenacetin, Acetylsalicylsäure oder andere pharmazeutisch aktive Stoffe in die Komprimate eingeschlossen werden. Die pharmazeutisch aktiven Wirkstoffe sind in einer Menge vorzusehen, die den erwünschten pharmazeutischen Effekt bewirken. Die schonende Verarbeitbarkeit der Komprimate macht die erfindungsgemäßen Komprimate besonders geeignet, pharmazeutisch aktive Wirkstoffe in den Mund- und Rachenraum zu verbringen.

Die Erfindung betrifft auch die mittels der vorgenannten Verfahren hergestellten Komprimate, insbesondere in Form von Lutsch-, Brause- oder Kautabletten.

Weitere vorteilhafte Ausgestaltungen der Erfindung ergeben sich aus den Unteransprüchen.

Die folgenden Beispiele erläutern die Erfindung in Einzelheiten.

### Referenz-Beispiel 1: Herstellung von Agglomeraten

Als Edukte wurde Isomalt (nahezu äquimolares Gemisch aus 1,1-GPM und 1,6-GPS) sowie eine Isomalt-Variante (Zusammensetzung: 1,1-GPM ≈ 51%; 1,6-GPS ≈ 38%; Sorbit ≈ 4%; Mannit 2%; sonstige ≈ 5%, jeweils Gew.-%, auf Trockensubstanz) eingesetzt.

Die Sprühtrocknung fand in einem Sprühbandtrockner statt, der einen Sprühturm und ein unter dem Sprühturm angeordnetes Gewebeband umfaßt.

Am Kopfende des Sprühturms wird eine 60 bis 70 Gew.-%ige Lösung der Edukte (bezogen auf Trockensubstanz) (Temperatur der Produktlösung: 65°C bis 80°C) und vermahlenes Trockenprodukt (Körnung 100 bis 300 µm) so zugeführt, daß eine möglichst vollständige Vermischung von Lösung und Pulver stattfindet. Der Sprühdruck der Edukt-Lösung betrug 120 bis 180 bar. Das Mengenverhältnis Pulver zu Edukt-Lösung betrug 3:1. Im Gleichstrom dazu wird Trocknungsluft mit einer Temperatur von 140°C bis 160°C zugeführt. Während der Fallzeit im Sprühturm verdampft das Wasser der Edukt-Lösung und eine aus dem Agglomerat gebildete Produktschicht wird auf dem Gewebeband abgeschieden. Das Gewebeband transportiert das Agglomerat in eine sich anschließende Nachtrocknungs- und Kühlzone, die mit einer Nachtrocknungs- und Kühlluft mit einer Temperatur von 20°C bis 60°C beaufschlagt wird. Das Produkt verbleibt in dem Nachtrocknungs- und Kühlbereich für 15 bis 25 min. und weist eine Temperatur von 25°C bis 35°C nach Verlassen des Kühlbereichs auf.

Gegebenenfalls kann eine Nachkristallisation in einer Trommel durchgeführt werden, vorzugsweise mit einer mittleren Verweilzeit von 1,5 bis 2,5 Stunden. Anschließend wird das Produkt zum Teil gemahlen und kontinuierlich als feines Pulver mit einem Partikeldurchmesser von 30 bis 300 µm zum Sprühturm zurückgeführt und dort in Kontakt mit eingesprühter Edukt-Lösung gebracht. Ein anderer Teil wird ausgeschleust, abgesackt und weiterverarbeitet.

Die so erhaltenen Agglomerate werden mittels einer Taumelsiebmaschine der Firma Allgaier mit einer Siebbelegung 0,8 mm bis 0,1 mm fraktioniert. Es wurden Siebfraktionen folgender Körnung hergestellt:
0,10 bis 0,20 mm
0,20 bis 0,30 mm
0,30 bis 0,40 mm
0,40 bis 0,50 mm
0,50 bis 0,63 mm
0,10 bis 0,63 mm.

In der nachstehenden Tabelle werden diese Fraktionen enthaltenden Komprimatrezepturen mit 01 - 02, 02 - 03, 03 - 04, 04 - 05, 05 - 06, 01 - 06 bezeichnet.

Die erhaltenen Fraktionen werden nach folgender Rezeptur zu verpreßbaren Komprimatmischungen zubereitet.

### Rezeptur:

| | |
|---|---|
| Isomalt oder Isomalt-Variante | 98,41 % |
| Mg-Stearat: | 0,50 % |
| Menthol L | 0,30 % |
| Aroma-Pfefferminz | 0,80 % |
| Acesulfam-K | 0,15 % |
| Aspartam | 0,15 % |

Alle Angaben in Gew.-%, bezogen auf Gesamttrockengewicht des Komprimates.

Die nachfolgende Tabelle I zeigt chemisch-physikalische Parameter der eingesetzten Komprimat-Mischungen auf.

**Tabelle I**

| Rezeptur | Wassergehalt | d05 | d95 | d | n | Schüttdichte | Stampfdichte | Rieselzeit |
|---|---|---|---|---|---|---|---|---|
| | % | mm | mm | mm | | g/cm³ | g/cm³ | s |
| 1. Isomalt 01-02 | 2,5 | 0,33 | 0,15 | 0,27 | 5,3 | 0,59 | 0,6 | 2,6 |
| 1. Isomalt 02-03 | 2,5 | 0,36 | 0,22 | 0,32 | 8,2 | 0,54 | 0,55 | 2,9 |
| 1. Isomalt 03-04 | 2,5 | 0,45 | 0,25 | 0,38 | 6,9 | 0,53 | 0,53 | 3,0 |
| 1. Isomalt 04-05 | 2,5 | 0,5 | 0,3 | 0,44 | 8,1 | 0,49 | 0,49 | 3,0 |
| 1. Isomalt 05-06 | 2,5 | 0,5 | 0,3 | 0,44 | 8,1 | 0,49 | 0,49 | 2,8 |
| 1. Isomalt 01-06 | 2,5 | 0,46 | 0,18 | 0,36 | 4,4 | 0,52 | 0,53 | 3,5 |
| 2. Isomalt 01-02 | 2,6 | 0,35 | 0,17 | 0,29 | 5,6 | 0,56 | 0,56 | 3,6 |
| 2. Isomalt 02-03 | 2,5 | 0,39 | 0,2 | 0,33 | 6,1 | 0,54 | 0,55 | 4,6 |
| 2. Isomalt 03-04 | 2,6 | 0,48 | 0,28 | 0,41 | 7,3 | 0,52 | 0,53 | 3,2 |
| 2. Isomalt 04-05 | 2,6 | 0,5 | 0,34 | 0,45 | 10,1 | 0,49 | 0,49 | 3,0 |
| 2. Isomalt 05-06 | 2,8 | 0,6 | 0,38 | 0,53 | 8,7 | 0,47 | 0,47 | 11,7* |
| 2. Isomalt 01-06 | 2,6 | 0,45 | 0,23 | 0,38 | 6,1 | 0,53 | 0,54 | 2,9 |
| | | | | | | | | |
| Tabelle (1. Isomalt | (ca. äquimolares Verhältnis GPM und GPS); | | | | | | | |
| 2. Isomalt | (ca. 51% 1,1-GPM, ca. 38% 1,6-GPS, ca. 4% Sorbit, ca. 2% Mannit, ca. 5% sonstige)) | | | | | | | |

| | | | | | | | | |
|---|---|---|---|---|---|---|---|---|
| Bestimmung der Rieselfähigkeit und der Rieselzeit nach DIN 53194 und DIN 53492 Art der Düse zur Bestimmung der Rieselfähigkeit: 15 mm Durchmesser : *, sonst 25 mm Durchmesser. Bestimmung der Schütt- und Stampfdichtigkeit nach DIN 53194 | | | | | | | | |

Die vorgenannten Mischungen für die Komprimat-Versuche wurden im Pflugscharmischer der Firma Lödige hergestellt. Die Mischzeit lag bei 1,5 min. Die Zudosierung der Einzelkomponenten erfolgte über eine Öffnung in der Deckelklappe des Mischers. Die Induzierung der flüssigen Komponate (Pfefferminz-Aroma) erfolgte über eine Verdüse-Einrichtung. Nach Beendigung des Mischvorganges wurden die Mischungen in PE-Säcken von je 5 kg abgefüllt und verschweißt.

Anschließend wurden mit den so erhaltenen Mischungen mittels einer FETTE PT 2090 Rundläuferpresse runde Tabletten mit einem Durchmesser von 18 mm und Facette, einer Steghöhe von 0,35 bis 0,37 mm und einem Gewicht zwischen 850 mg bis 1000 mg hergestellt.

### Referenz-Beispiel 2: Bruchkraftvergleiche

Die folgende Tabelle II zeigt einen Bruchkraftvergleich zwischen Isomalt FE (Stand der Technik) und den Komprimaten aus Isomalt (1. Isomalt 03 - 04) und einer Isomaltvariante (2. Isomalt 03 - 04) (vgl. Tabelle I). Es zeigt sich, daß bei gleicher Preßkraft erheblich stabilere Komprimate erhalten werden können, wenn Agglomerate gemäß des Referenz-Beispiels 1 eingesetzt werden.

### Bruchkraftvergleich Isomalt Typ FE und agglomerierte Typen

**Tabelle II**

| | Isomalt Typ FE* (d 95 ca. 300 µm) | 1. Isomalt 03-04 (d 95 = 0,25 µm) | 2. Isomalt 03-04 (d 95 = 0,28 µm) |
|---|---|---|---|
| Bruchkraft in N | 62 | 201 | 220 |
| Preßkraft in kN | 50 | 50 | 50 |

| | | | |
|---|---|---|---|
| *Stand der Technik | | | |

## Patentansprüche

1. Verfahren zur Herstellung eines agglomerierten, fließfähigen Produktes aus Isomaltulose, wobei eine Isomaltulose-Lösung oder Suspension mit einem Trockensubstanzgehaft von 30 bis 70 Gew.-% unter Zufuhr von Trockenluft in ein Pulver von Isomaltulose gesprüht wird; wobei das Mengenverhältnis von Pulver zu Edukt-Lösung oder -Suspension 1:1 bis 3,5:1 beträgt, wobei die Trocknungsluft eine Temperatur von 120 bis 180° C aufweist und wobei das so erhaltene agglomerierte Produkt einer Nachtrocknung und Kühlung unterzogen sowie ein agglomeriertes, fließfähiges Produkt erhalten wird.

2. Verfahren nach Anspruch 1, wobei im Anschluss an die Kühlung des nachgetrockneten Produktes eine Nachkristallisation stattfindet.

3. Verfahren nach einem der vorhergehenden Ansprüche, wobei im Anschluss an die Kühlung oder die Nachkristallisation zumindest ein Teil des erhaltenen agglomerierten, fließfähigen Produktes vermahlen wird.

4. Verfahren nach Anspruch 3, wobei ein Teil des vermahlenen Pulvers als Edukt in das Verfahren gemäß Anspruch 1, 2 oder 3 eingeführt wird.

5. Verfahren nach einem der vorhergehenden Ansprüche, wobei die Isomaltulose-haltige Lösung oder Suspension eine Temperatur von 50°C bis 90°C aufweist.

6. Verfahren nach einem der vorhergehenden Ansprüche, wobei die Isomaltulose-haltige Lösung oder Suspension unter einem Sprühdruck von 100 bis 200 bar in das Pulver gesprüht wird.

7. Verfahren nach einem der vorhergehenden Ansprüche, wobei das Pulver eine Körnung von 50 bis 400 µm aufweist.

8. Verfahren nach einem der vorhergehenden Ansprüche, wobei die Nachtrocknung und die Kühlung unter Zufuhr von Luft mit einer Temperatur von 20°C bis 80°C stattfinden.

9. Verfahren nach einem der vorhergehenden Ansprüche, wobei die Nachtrocknung und die Kühlung über einen Zeitraum von 10 bis 30 min. stattfinden.

10. Verfahren nach einem der vorhergehenden Ansprüche, wobei die Nachkristallisation über einen Zeitraum von 1 bis 4 Stunden stattfindet.

11. Verfahren nach einem der vorhergehenden Ansprüche, wobei die Isomaltulose-haltige Lösung oder Suspension zusammen mit Hilfs-, Zusatz-, Wirkstoffen, Trennmitteln, Gleitmitteln, Geschmackstoffen, Aromastoffen, Süßstoffen, lebensmittelverträglichen Säuren, Sprengmitteln oder Farbstoffen sprühgetrocknet wird.

12. Agglomerat, herstellbar nach einem der Verfahren der vorhergehenden Ansprüche.

13. Verfahren zur Verstellung eines Komprimates, wobei ein Verfahren nach einem der Ansprüche 1 bis 11 durchgeführt und das erhaltene Agglomerat zu einem Komprimat verpreßt wird.

14. Verfahren nach Anspruch 13, wobei dem Agglomerat vor dem Verpressen Hilfs-, Zusatz-, Wirkstoffe, Trennmittel, Gleitmittel, Geschmackstoffe, Aromastoffe, Süßstoffe, lebensmittelverträgliche Säuren, Sprengmittel oder Farbstoffe zugefügt werden.

15. Komprimat, herstellbar nach einem der Verfahren der Ansprüche 13 oder 14.

## Claims

1. Method for producing an agglomerated product, which is capable of flowing, from isomaltulose, wherein a solution or suspension containing isomaltulose with a dry solids content of 30 to 70 wt% is sprayed with a supply of dry air into a powder of isomaltulose, with the quantity ratio of powder to educt solution or suspension amounting to 1:1 to 3.5:1, wherein the dry air has a temperature of 120 to 180 °C, and wherein the resulting agglomerated product is subjected to a secondary drying and cooling, and an agglomerated product, which is capable of flowing, is obtained.

2. Method according to claim 1, wherein a secondary crystallisation takes place following the cooling of the secondarily dried product.

3. Method according to any one of the preceding claims, wherein at least a portion of the resulting agglomerated product, which is capable of flowing, is ground following the cooling or the secondary crystallisation.

4. Method according to claim 3, wherein a portion of the ground powder is introduced into the method according to claim 1, 2 or 3 in the form of an educt.

5. Method according to any one of the preceding claims, wherein the solution or suspension containing isomaltulose has a temperature of 50 °C to 90 °C.

6. Method according to any one of the preceding claims, wherein the solution or suspension containing isomaltulose is sprayed into the powder at a spray pressure of 100 to 200 bar.

7. Method according to any one of the preceding claims, wherein the powder has a particle size of 50 to 400 µm.

8. Method according to any one of the preceding claims, wherein the secondary drying and the cooling take place with a supply of air at a temperature of 20 °C to 80 °C.

9. Method according to any one of the preceding claims, wherein the secondary drying and the cooling take place over a period of 10 to 30 minutes.

10. Method according to any one of the preceding claims, wherein the secondary crystallisation takes place over a period of 1 to 4 hours.

11. Method according to any one of the preceding claims, wherein the solution or suspension containing isomaltulose together with auxiliary substances, additives, active ingredients, parting compounds, lubricants, flavour enhancers, flavouring agents, sweeteners, food-compatible acids, disintegrants or colouring agents, is spray-dried.

12. Agglomerate, producible according to any one of the methods of the preceding claims.

13. Method for producing a compressed product, wherein a method according to any one of claims 1 to 11 is carried out, and the resulting agglomerate is pressed to form a compressed product.

14. Method according to claim 13, wherein auxiliary substances, additives, active ingredients, parting compounds, lubricants, flavour enhancers, flavouring agents, sweeteners, food-compatible acids, disintegrants or colouring agents are added to the agglomerate before pressing.

15. Compressed product, producible according to any one of the methods of claim 13 or 14.

## Revendications

1. Procédé pour produire un produit aggloméré fluide d'isomaltulose, dans lequel une solution ou une suspension contenant de l'isomaltulose avec une teneur en substance sèche de 30 à 70 % en poids est vaporisée dans une poudre d'isomaltulose en ajoutant de l'air sec, le ratio entre la quantité de la poudre et la quantité de la solution de base ou la suspension de base étant de 1 : 1 à 3,5 : 1, dans lequel l'air de séchage a une température de 120 °C à 180 °C, et dans lequel le produit aggloméré ainsi obtenu est soumis à un procès de séchage de suite et de refroidissement et on obtient un produit aggloméré fluide.

2. Procédé selon la revendication 1, dans lequel une cristallisation secondaire a lieu suite au procès de séchage de suite et de refroidissement du produit.

3. Procédé selon l'une des revendications précédentes, dans lequel au moins une partie du produit aggloméré fluide qu'on vient d'obtenir est moulue suite au refroidissement ou à la cristallisation secondaire.

4. Procédé selon la revendication 3, dans lequel une partie de la poudre moulue est introduite comme matière de base dans le procédé conformément à la revendication 1, 2 ou 3.

5. Procédé selon l'une des revendications précédentes, dans lequel la solution ou la suspension contenant de l'isomaltulose a une température de 50 °C à 90 °C.

6. Procédé selon l'une des revendications précédentes, dans lequel la solution ou la suspension contenant de l'isomaltulose est vaporisée dans la poudre à une pression de vaporisation de 100 à 200 bar.

7. Procédé selon l'une des revendications précédentes, dans lequel les particules de la poudre ont une taille de 50 à 400 µm.

8. Procédé selon l'une des revendications précédentes, dans lequel le procès de séchage de suite et de refroidissement a lieu en ajoutant de l'air d'une température de 20 °C à 80 °C.

9. Procédé selon l'une des revendications précédentes, dans lequel le procès de séchage de suite et de refroidissement a lieu sur une période de 10 à 30 minutes.

10. Procédé selon l'une des revendications précédentes, dans lequel la cristallisation secondaire a lieu sur une période de 1 à 4 heures.

11. Procédé selon l'une des revendications précédentes, dans lequel la solution ou la suspension contenant de l'isomaltulose est séchée par vaporisation, conjointement avec des matières auxiliaires, des additifs, des substances actives, des agents séparateur, des lubrifiants, des exhausteurs de goût, des agents aromatisants, des agents édulcorants, des acides inoffensifs pour les aliments, des délitants ou des agents colorants.

12. Aggloméré, productible selon l'un des procédés des revendications précédentes.

13. Procédé pour produire un produit comprimé, dans lequel un procédé selon l'une des revendications 1 à 11 est exécuté, et l'aggloméré résultant est comprimé pour obtenir un produit comprimé.

14. Procédé selon la revendication 13, dans lequel des matières auxiliaires, des additifs, des substances actives, des agents séparateur, des lubrifiants, des exhausteurs de goût, des agents aromatisants, des agents édulcorants, des acides inoffensifs pour les aliments, des délitants ou des agents colorants sont ajoutés à l'aggloméré avant le pressage.

15. Produit comprimé, productible selon l'un des procédés des revendications 13 ou 14.
